(19) Europäisches Patentamt
European Patent Office
Office européen des brevets

(11) **EP 4 741 368 A1**

(12) **EUROPEAN PATENT APPLICATION**

(43) Date of publication:
**13.05.2026 Bulletin 2026/20**

(21) Application number: **24306888.9**

(22) Date of filing: **11.11.2024**

(51) International Patent Classification (IPC):
**C07C 7/04** (2006.01)    **C07C 15/46** (2006.01)

(52) Cooperative Patent Classification (CPC):
(C-Sets available)
**C07C 7/04**    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Technip Energies France
92741 Nanterre Cedex (FR)**

(72) Inventor: **Hubbell, Douglas Stuart
92741 Nanterre Cedex (FR)**

(74) Representative: **Edson, Russell Gregory et al
Withers & Rogers LLP
2 London Bridge
London SE1 9RA (GB)**

(54) **STYRENE RESIDUE RECYCLE PROCESSES AND RELATED SYSTEMS**

(57)    Systems and methods for purifying styrene monomer may include a styrene prefractionation system, a styrene finishing system, and a residue recycle processer. The residue recycle processer may heat and separate a residue stream from the styrene finishing system to produce a residue recycle stream with lower concentrations of $C_9$ and $C_{10}$ aromatic hydrocarbons therein compared to the residue stream. The residue recycle stream may be recycled back to the styrene prefractionation system. Such systems and methods for purifying styrene monomer may have less energy consumption, lower polymerization inhibitor dosage, less air pollution, and/or less equipment fouling.

FIG. 1

**(Cont. next page)**

(52) Cooperative Patent Classification (CPC): (Cont.)

C-Sets
**C07C 7/04, C07C 15/46**

# EP 4 741 368 A1

## Description

<u>Technical Field</u>

The present disclosure relates generally to chemical processing and, more particularly (although not necessarily exclusively), to purifying styrene monomer with less energy consumption, lower polymerization inhibitor dosage, less air pollution, and/or less equipment fouling.

<u>Background</u>

Styrene monomer manufacturing produces a crude styrene stream from a dehydrogenation section. The compounds lighter than styrene may be stripped in one or more distillation columns, hereinafter collectively referred to as a styrene prefractionation system. The remaining stream, including styrene monomer and less volatile impurities, is fed to a styrene finishing system, which typically starts with a distillation finishing column that has a reboiler and condenser, operates under vacuum, and recovers a final styrene monomer product as distillate. The heavier impurities in this stream include $C_9$ aromatic hydrocarbons (such as propylbenzene and methylstyrene isomers), $C_{10}$ aromatic hydrocarbons (such as diethylbenzene and divinylbenzene isomers), multiple-ring aromatics that are less volatile than styrene (primarily condensation products of styrene, including stilbene), and styrene polymer. One or more types of polymerization inhibitor compounds are added typically to the styrene prefractionation system to reduce the loss of styrene to polymer (polystyrene) during distillation. The inhibitors that are slow to react but provide extended inhibition time are also referred to as retarders. These inhibitors typically have nitrogen in their molecules. Because the net residue of a styrene process may be burned to recover its fuel value, use of these polymerization inhibitors may result in emissions of harmful nitrogen oxides to the environment.

The bottoms stream from the distillation finishing column is typically partially vaporized in a residue reboiler (e.g., a steam-heated exchanger) and fed to a residue flash drum where the vapor is separated from the liquid and fed back to the distillation finishing column. This residue flash drum liquid contains most of the low-volatility (heavy) by-products of the styrene process, as well as residual styrene, typically at a concentration of about 5.0 wt%. A portion of this liquid from the residue flash drum may be optionally recycled to the styrene prefractionation system upstream of the styrene finishing system to reuse residual active polymerization inhibitors, especially the retarder. The rest of the liquid is the net heavy by-product stream (residue) of the styrene process.

<u>Summary</u>

One or more embodiments include a system comprising: a styrene finishing system configured to receive a first portion of a styrene-rich stream from a styrene prefractionation system and produce a styrene monomer product, a residue stream, and a net heavy by-product stream; a residue recycle processor configured to process a second portion of the styrene-rich stream from the styrene prefractionation system and the residue stream from the styrene finishing system to produce an overhead vapor stream and a residue recycle stream; and wherein the styrene finishing system is further configured to receive the overhead vapor stream; and wherein the styrene prefractionation system is configured to receive the residue recycle stream from the residue recycle processer.

One or more embodiments include the system of the previous paragraph, wherein the residue recycle stream comprises 15 wt% or less of styrene and 0.10 wt% or greater of a polymerization inhibitor.

One or more embodiments include the system of any previous paragraph, wherein the second portion of the styrene-rich stream comprises 90 wt% or greater of styrene.

One or more embodiments include the system of any previous paragraph, wherein the residue recycle processor comprises a residue recycle reboiler and a residue recycle drum, wherein the residue recycle reboiler is configured to receive and heat the second portion of the styrene-rich stream from the styrene prefractionation system and the residue stream from the styrene finishing system as a combined stream to produce a partially vaporized stream, wherein the residue recycle drum is configured to receive and separate the partially vaporized stream to produce the overhead vapor stream and a bottoms liquid stream, and wherein at least a portion of the bottoms liquid stream is the residue recycle stream.

One or more embodiments include the system of any previous paragraph, wherein the styrene finishing system comprises one or more distillation finishing columns, one or more reboilers, and one or more drums.

One or more embodiments include the system of any previous paragraph, wherein the overhead vapor stream is a first overhead vapor stream, wherein the bottoms liquid stream is a first bottoms liquid stream, and wherein the styrene finishing system comprises: a distillation finishing column configured to receive the first portion of the styrene-rich stream from the styrene prefractionation system and the first overhead vapor stream from the residue recycle processor and to produce a second overhead vapor stream and a second bottoms liquid stream; and a residue reboiler and a residue flash drum in series configured to receive the second bottoms liquid stream and to produce a third overhead vapor stream and a third bottoms liquid stream, wherein the system is configured to split the third bottoms liquid stream to produce the residue stream and the net heavy by-product stream.

One or more embodiments include a method comprising: purifying a first portion of a styrene-rich stream from a styrene prefractionation system in a styrene finishing system to produce a styrene monomer product, a residue stream, and a net heavy by-product stream; heating then separating at least a portion of the residue stream using a second portion of the styrene-rich stream as a stripping agent to yield an overhead vapor stream and a bottoms liquid stream; recycling the overhead vapor stream to the styrene finishing system; and recycling at least a portion of the bottoms liquid stream as a residue recycle stream to the styrene prefractionation system.

One or more embodiments include the method of the previous paragraph, wherein the residue stream comprises 15 wt% or less of styrene and 0.10 wt% or greater of a polymerization inhibitor.

One or more embodiments include the method of any previous paragraph, wherein the second portion of the styrene-rich stream comprises 90 wt% or greater of styrene.

One or more embodiments include the method of any previous paragraph, wherein the second portion of the styrene-rich stream is up to 25 wt% of the styrene-rich stream.

One or more embodiments include the method of any previous paragraph, wherein a percent reduction of alpha-methylstyrene in the residue recycle stream compared to the residue stream is 50% or greater.

One or more embodiments include the method of any previous paragraph, wherein a percent reduction of divinylbenzene in the residue recycle stream compared to the residue stream is 40% or greater.

One or more embodiments include the method of any previous paragraph, wherein a weight ratio of the residue stream to the net heavy by-product stream is 0.5:1 to 10:1.

One or more embodiments include the method of any previous paragraph, wherein a weight ratio of the second portion of the styrene-rich stream to the residue stream is 0.1:1 to 10:1.

Brief Description of the Drawings

FIG. 1 illustrates a process flow diagram of an example system, according to at least some examples of the present disclosure.

FIG. 2 illustrates a process flow diagram of another example system, according to at least some examples of the present disclosure.

FIG. 3 illustrates a flow chart for a method for producing a styrene monomer product, according to at least some embodiments of the present disclosure.

Detailed Description

Certain aspects and features of the present disclosure relate to producing a higher quality residue recycle stream in a styrene finishing system by passing the residue recycle stream through a residue recycle processor to vaporize certain components before recycling the liquid back to the styrene prefractionation system. More specifically, the residue recycle processor may reduce the concentrations of $C_9$ and $C_{10}$ aromatics (e.g., alpha-methylstyrene (AMS) and divinylbenzene (DVB)) in the residue recycle stream that is conveyed back to the styrene prefractionation system.

In conventional systems, recycling residue back to the styrene prefractionation system allows for decreases the required dosage of one or more polymerization inhibitors. However, because $C_9$ and $C_{10}$ aromatics are typically in this residue recycle stream, their steady-state concentrations in the prefractionation and styrene finishing systems are higher because of the residue recycle. With higher amounts of $C_9$ aromatics in the feed to the styrene finishing system, typically AMS is the $C_9$ aromatic having the highest concentration, more total reboiler duty may be required by the styrene finishing system reboilers. As discussed more below, DVB, when reacted with styrene polymer, can form a crosslinked polymer that may foul equipment. Higher concentrations of DVB may make fouling more likely. Advantageously, the systems and methods of the present disclosure may remove certain unwanted compounds (e.g., DVB and AMS) in the residue recycle stream using a residue recycle processer. As illustrated herein, even with the addition of the residue recycle processer, the overall heat duty within the system can be reduced.

As noted above, DVB is a crosslinking agent for polystyrene, and crosslinked polystyrene does not dissolve in styrene and other aromatics, while uncrosslinked polystyrene does. The crosslinked or insoluble polymer may attach to metal surfaces and reduce the heat transfer efficiency of reboilers, may cause flow distributors to mal-distribute, and may foul the packing and/or trays inside the distillation columns in the styrene prefractionation and styrene finishing systems. The insoluble, crosslinked polymer, which can be very difficult to remove from equipment, may greatly reduce the effectiveness of the styrene prefractionation and styrene finishing systems. Removal of the insoluble, crosslinked polymer often requires shutting down the process and physically entering the equipment. In conventional systems, when appreciable concentrations of DVB are recycled back to the styrene prefractionation system from the styrene finishing system, the likelihood of serious fouling increases. In some instances, recycling residue is omitted in conventional operations to minimize fouling. Advantageously, the systems and methods of the present disclosure may significantly reduce the concentration of DVB in the stream recycled back to the styrene prefractionation system, which may greatly mitigate the potential for fouling. Further, because of the lower DVB concentration in the stream recycled back to the styrene prefractionation system, significantly more residue flow may be recycled. Because the residue stream also includes polymerization inhibitors, the increased recycle volume may reduce the overall dosage of polymerization inhibitors.

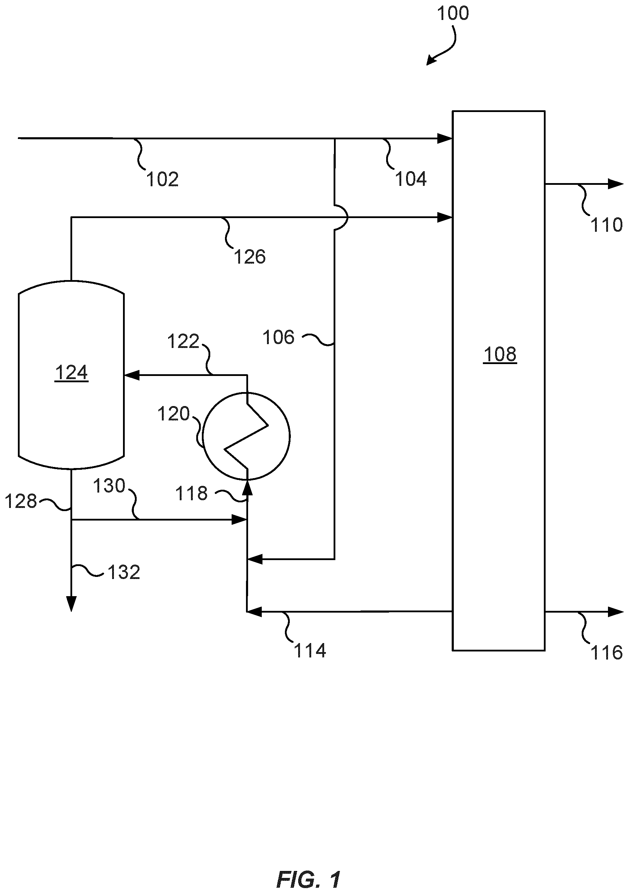

FIG. 1 illustrates a process flow diagram of a system 100, according to at least some examples of the present disclosure. The system 100 may be used as a portion of a styrene purification process for the production of styrene monomer product. The illustration provides a flow diagram where other equipment may be incorporated for proper operation of the system 100. For example, additional equipment like sensors, valves, pumps, and the like may be included within the flow diagram but are omitted from the figures for simplicity. Additionally, other flow streams may be included depending on the configuration of the system and its integration with other equipment and/or portions of other systems.

The system may include a styrene finishing system 108 that produces a styrene monomer product stream 110, a residue stream 114, and a net heavy by-product stream 116. The styrene finishing system 108 may include one or more distillation finishing columns, one or more reboilers, one or more condensers, optionally one or more drums, or any combination thereof.

The styrene finishing system 108 may be located downstream of a styrene prefractionation system that comprises one or more distillation columns that remove and recover compounds more volatile than styrene from the crude styrene from the dehydrogenation section of the styrene process. The system 100 of the present disclosure may advantageously include a residue recycle reboiler 120 and a residue recycle drum 124 to reduce the concentrations of $C_9$ and $C_{10}$ aromatics (e.g., AMS and DVB) in the residue recycle stream 132 relative to the residue stream 114.

The illustrated system 100 receives a styrene-rich stream 102 from the styrene prefractionation system. The styrene-rich stream 102 includes styrene monomer, typically at 90 wt% or greater (e.g., 92 wt% or greater, or 95 wt% or greater), and impurities. Examples of heavy impurities (i.e., impurities that are less volatile than styrene) may include, but are not limited to, $C_9$ aromatics (e.g., propylbenzenes and methylstyrenes), $C_{10}$ aromatics (e.g., diethylbenzenes and divinylbenzenes), multiple-ring aromatics which are less volatile than styrene (primarily condensation products of styrene, including stilbene), styrene polymer, the like, and any combination thereof.

The styrene-rich stream 102 may be split with (i) the majority of the styrene-rich stream 102 (illustrated as a first portion 104 of the styrene-rich stream 102) being fed to the styrene finishing system 108 and (ii) a slip stream of the styrene-rich stream 102 (illustrated as a second portion 106 of the styrene-rich stream 102) being conveyed to a residue recycle processor (illustrated as a residue recycle reboiler 120 and a residue recycle drum 124). The second portion 106 of the styrene-rich stream 102 may be up to 25 wt% (e.g., up to 20 wt%, up to 15 wt%, up to 10 wt%, 0.1 wt% to 25 wt%, 0.5 wt% to 20 wt%, or 1 wt% to 15 wt%) of the styrene-rich stream 102.

The styrene finishing system 108 receives the first portion 104 of the styrene-rich stream 102 and produces the styrene monomer product stream 110, the residue stream 114, and the net heavy by-product stream 116. The styrene finishing system 108 may further have input streams and output streams based on the configuration of the styrene finishing system 108. The residue stream 114 may include primarily heavy impurities and less than 15 wt% styrene. Of particular note may be the $C_9$ and $C_{10}$ aromatics, especially AMS and DVB, and polymerization inhibitors. Advantageously, the systems and methods of the present disclosure may treat the residue stream 114 to produce a residue recycle stream 132 with lower concentrations of the $C_9$ and $C_{10}$ aromatics, especially, AMS and DVB. The residue recycle stream 132 may be recycled back to the styrene prefractionation system.

A recycle ratio of the residue stream 114 to the net heavy by-product stream 116 (or the weight ratio of the residue stream 114 to the net heavy by-product stream 116) may range from 0.5:1 to 10:1 (e.g., 1:1 to 10:1, 0.5:1 to 8:1, 1:1 to 8:1, 0.5:1 to 6:1, or 1:1 to 6:1).

The residue stream 114 may include one or more polymerization inhibitors at 0.10 wt% or greater (e.g., 0.10 wt% to 20 wt%, 0.50 wt% to 10 wt%, or 1.0 wt% to 7 wt%), based on a total weight of the residue stream 114. For example, the residue stream 114 may include AMS at 0.5 wt% or greater (e.g., 0.5 wt% to 10 wt%, 0.5 wt% to 8 wt%, 1.0 wt% to 8 wt%, or 2 wt% to 7 wt%), based on a total weight of the residue stream 114. For example, the residue stream 114 may include DVB at 0.05 wt% or greater (e.g., 0.05 wt% to 10 wt%, 0.1 wt% to 5 wt%, or 0.1 wt% to 3 wt%), based on a total weight of the residue stream 114. One or both of the AMS and DVB may be present in the residue stream 114 at the forgoing concentrations.

The residue stream 114 may include styrene at 15 wt% or less (e.g., 0.1 wt% to 15 wt%, 0.5 wt% to 10 wt%, or 1.0 wt% to 7 wt%), based on a total weight of the residue stream 114.

A residue recycle processor configured to process a second portion 106 of the styrene-rich stream 102 from the styrene prefractionation system and the residue stream 114 from the styrene finishing system to produce an overhead vapor stream and a residue recycle stream. As illustrated, the second portion 106 of the styrene-rich stream 102 and the residue stream 114 may be mixed to produce a combined stream 118. The combined stream 118 may be further processed using the residue recycle processor to produce an overhead vapor stream 126 and the residue recycle stream 132.

Alternatively, the second portion 106 of the styrene-rich stream 102 may be heated before mixing with the residue stream 114. The resultant combined stream (not illustrated) may be separated to produce the overhead vapor stream 126 and the residue recycle stream 132.

The styrene in the second portion 106 of the styrene-rich stream 102 may be used as a stripping agent for more efficient removal of $C_9$ and $C_{10}$ aromatics in the residue recycle processer. A weight ratio of the second portion 106 of the styrene-rich stream 102 relative to residue stream 114 may range from 0.1:1 to 10:1 (e.g., 0.1:1 to 8:1, 0.3:1 to 8:1, or 0.3:1 to 5:1).

The residue recycle processor of FIG. 1 is illustrated as a residue recycle reboiler 120 and a residue recycle drum 124 as separate pieces of equipment. However, any suitable configuration of equipment suitable for receiving the second portion of the styrene-rich stream 106 and the residue stream 114 and producing the overhead vapor stream 126 and the residue recycle stream 132 may be used as the residue recycle processer. Accordingly, there are multiple configurations that could be implemented for the residue recycle processer. For example, the residue recycle processor may include a residue recycle reboiler and drum integrated into a single piece of equipment.

In the illustrated example, the combined stream 118 may be fed to the residue recycle reboiler 120 of the residue recycle processer and heated to produce a partially vaporized stream 122. The partially vaporized stream 122 may then be fed to the residue recycle drum 124 of the residue recycle processor to remove AMS, other $C_9$ aromatics, and DVB as part of an overhead vapor stream 126. At least a portion of the overhead vapor stream 126 may be recycled back to the styrene finishing system 108.

The residue recycle drum 124 may operate at a pressure of 300 mm Hg absolute or less (e.g., 250 mm Hg absolute or less, 200 mm Hg absolute or less, 50 mm Hg absolute to 300 mm Hg absolute, 100 mm Hg absolute to 250 mm Hg absolute, or 100 mm Hg absolute to 200 mm Hg absolute). The residue recycle drum 124 may operate at a temperature of 180°C or less (e.g., 125°C to 180°C, 125°C to 175°C, or 140°C to 165°C).

The residue recycle drum 124 also produces a bottoms liquid stream 128. Optionally, a first portion of the bottoms liquid stream 128, illustrated as recirculation stream 130, may be recirculated back to the residue recycle reboiler 120. Recycling the recirculation stream 130 back to the residue recycle reboiler 120 is optional but may advantageously improve the performance of the residue recycle reboiler 120. The recirculation stream 130 may be mixed with the residue stream 114 and the second portion 106 of the styrene-rich stream 102 (in any order of mixing) to produce the combined stream 118.

A second portion of bottoms liquid stream 128 from the residue recycle drum 124 may be recycled back to the styrene prefractionation system as the residue recycle stream 132. Alternatively, all of the bottoms liquid stream 128 may be the residue recycle stream 132.

Comparing the residue recycle stream 132 to the residue stream 114, the percent reduction of a compound (e.g., AMS or DVB) in the residue recycle stream 132 can be calculated as follows where each flowrate is weight per unit of time, such as kg/hr.

$$\% \, red. \, of \, cmpd. = 100 - \left[ \left( \frac{flowrate \, of \, cmpd. \, in \, residue \, recycle \, stream \, 132}{flowrate \, of \, cmpd. \, in \, residue \, stream \, 114} \right) * 100 \right]$$

The percent reduction of AMS in the residue recycle stream 132 compared to the residue stream 114 may be 50% or greater (e.g., 60% or greater, 70% or greater, 80% or greater, 90% or greater, 50% to 99%, 60% to 99%, 70% to 99%, 80% to 99%, or 90% to 99%).

The percent reduction of DVB in the residue recycle stream 132 compared to the residue stream 114 may be 40% or greater (e.g., 50% or greater, 60% or greater, 70% or greater, 40% to 99%, 50% to 99%, 60% to 99%, or 70% to 99%).

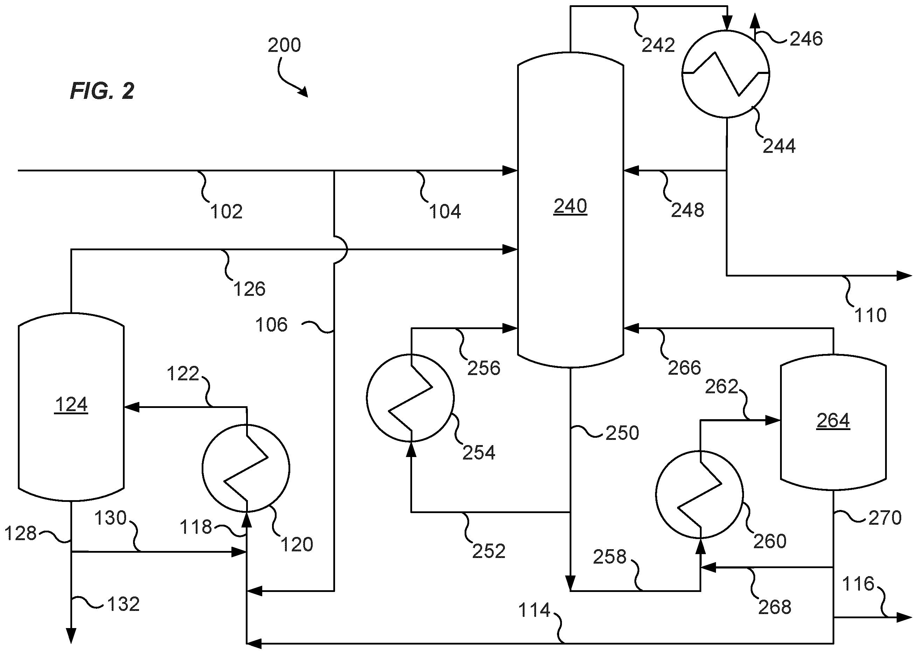

FIG. 2, with continued reference to FIG. 1, illustrates a process flow diagram of a system 200, according to at least some examples of the present disclosure. Where the same reference numbers from FIG. 1 are used in FIG. 2, it is understood that the description of FIG. 1, including concentrations, flows, operating conditions, and the like, apply to the example system 200 of FIG. 2. Further, as with FIG. 1, additional equipment and flow streams may be included in FIG. 2 may be incorporated for proper operation of the system 200.

The system 200 may be used as a portion of a styrene purification process for the production of styrene monomer product. A styrene prefractionation system that comprises one or more distillation columns may be upstream of the illustrated system. The illustrated system 200 receives a styrene-rich stream 102 from the styrene prefractionation system. The styrene-rich stream 102 may be split into the first portion 104 of the styrene-rich stream 102 and the second portion 106 of the styrene-rich stream 102, where the first portion 104 of the styrene-rich stream 102 may be fed to the styrene finishing system, as describe above in FIG. 1.

The styrene finishing system in the illustrated example includes a distillation finishing column 240, a condenser 244, two reboilers 254 and 260, and a flash drum 264. Other configurations are also suitable. As discussed previously, the styrene finishing system may include one or more distillation finishing columns, one or more reboilers, one or more condensers, optionally one or more drums, or any combination thereof.

In the illustrated example, the distillation finishing column 240 receives the first portion 104 of the styrene-rich stream 102. The distillation finishing column 240 may separate the first portion 104 of the styrene-rich stream 102 into an overhead vapor stream 242 and a bottoms liquid stream 250. The distillation finishing column 240 internals may include either all trays, all packing, or a combination of trays and packing.

The overhead vapor stream 242 may be fully or partially condensed in a finishing column condenser 244, thereby producing a liquid distillate stream (e.g., styrene monomer product stream 110), a column reflux stream 248, and typically a small vapor distillate stream 246.

The bottoms liquid stream 250 from the distillation finishing column 240 may be split. A first portion of the bottoms liquid stream 250, illustrated as recirculation stream 252, may flow to a finishing column reboiler 254. The finishing column reboiler 254 may heat the recirculation stream 252, which, as stream 256, provides heat to the distillation finishing column 240.

A second portion 258 of the bottoms liquid stream 250 flows to a heated exchanger (e.g., a steam-heated exchanger), referred to herein as a residue reboiler 260. The residue reboiler 260 may heat the second portion 258 of the bottoms liquid stream 250 to yield a partially vaporized bottoms stream 262. The partially vaporized bottoms stream 262 from the residue reboiler 260 may be fed to a residue flash drum 264, which separates the partially vaporized bottoms stream 262 into a bottoms liquid stream (illustrated as residue stream 270) and an overhead vapor stream 266.

The distillation finishing column 240 and the residue flash drum 264 may operate at deep vacuum, which may result in low operating temperatures that minimize i) the loss of styrene polymerizing to form polymer, ii) the dosages of polymerization inhibitors, and iii) the combined system reboiler duty. For example, the distillation finishing column 240 may be operated with the column top at 30 mm Hg absolute to 60 mm Hg absolute (e.g., 30 mm Hg absolute to 50 mm Hg absolute, 35 mm Hg absolute to 55 mm Hg absolute, or 40 mm Hg absolute to 60 mm Hg absolute).

The distillation finishing column 240 may operate at a bottom temperature of 65°C to 120°C (e.g., 70°C to 120°C, 80°C to 115°C, or 80°C to 105°C). The residue flash drum 264 may operate at a temperature ranging from 125°C to 180°C (e.g., 125°C to 175°C, 125°C to 170°C, or 130°C to 160°C).

Referring back to FIG. 2, the overhead vapor stream 266 from the residue flash drum 264 may be returned to the distillation finishing column 240 and may provide heat to the distillation finishing column 240. The residue stream 270 may be split into two or more streams. In the illustrated system 200, the residue stream 270 is split into a recirculation stream 268, a residue stream 114, and a net heavy by-product stream 116, which is the net residue of the styrene process.

The recirculation stream 268 may be fed back to the residue reboiler 260. The inclusion of the recirculation stream 268 is optional but may advantageously improve the performance of the residue reboiler 260.

The remaining equipment and stream flows are described relative to FIG. 1 and apply here to FIG. 2.

The residue recycle drum 124 may operate at a higher pressure than the point in a distillation finishing column 240 where the overhead vapor stream 126 is fed. The location along the distillation finishing column 240 where the overhead vapor stream 126 is introduced may be optimized. The residue recycle drum 124 may operate at a temperature greater than the operating temperature of the bottom of the distillation finishing column 240.

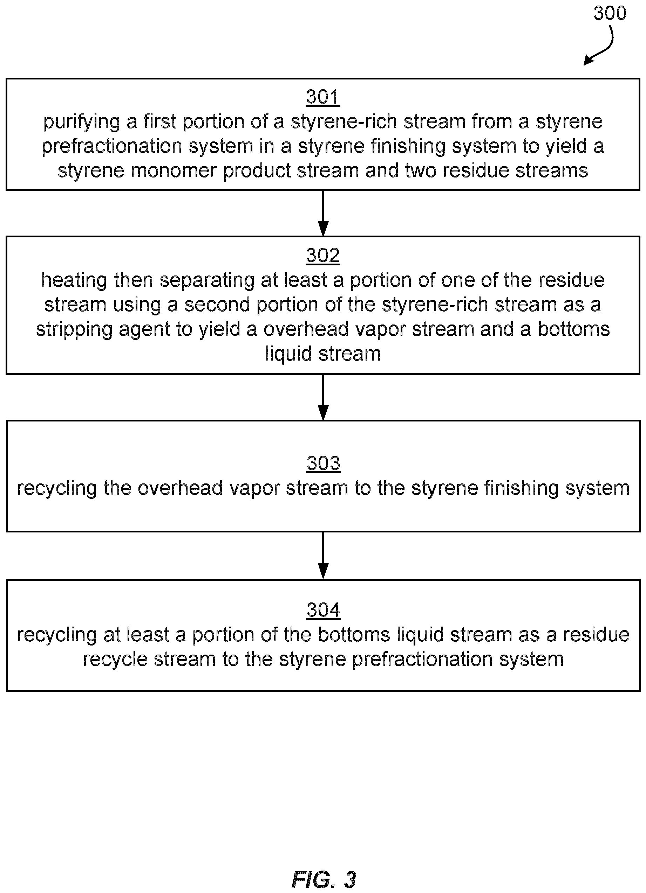

FIG. 3 illustrates a flow chart for a method 300 for producing a styrene monomer product according to at least some embodiments of the present disclosure. The operating conditions, stream compositions, and the like described relative to FIGS. 1 and 2 apply to the methods of the present disclosure including the example method of FIG. 3.

Step 301 may include purifying a first portion of a styrene-rich stream from a styrene prefractionation system in a styrene finishing system to yield a styrene monomer product stream, two residue streams, and possibly a vapor distillate stream (e.g., as illustrated in FIG. 2).

Step 302 may include heating then separating one of the two residue streams using a second portion of the styrene-rich stream as a stripping agent to yield an overhead vapor stream and a bottoms liquid stream.

Step 303 may include recycling the overhead vapor stream to the styrene finishing system.

Step 304 may include recycling at least a portion of the bottoms liquid stream as a residue recycle stream to the styrene prefractionation system.

Methods of the present disclosure may include additional steps, including those described relative to FIG. 2. For example, a distillation finishing column may be used to achieve the heating then separating in Step 301. A portion of a bottoms liquid from the distillation finishing column may be recirculated back to the distillation finishing column. Further, a residue flash drum downstream configured to receive another portion of the bottoms liquid from the distillation finishing column may be further used in the heating then separating in Step 301. Before the heating and separating in Step 302, a portion of a bottoms liquid from the residue flash drum may be recirculated back to and combined with the portion of the bottoms liquid from the distillation finishing column flowing to the residue flash drum. In another example, an overhead vapor from the residue flash drum may be recycled to the distillation finishing column used to achieve the heating then separating in Step 301.

Examples

Simulations for various systems including either a comparative styrene finishing system and an inventive styrene finishing system were run using a commercially available computer process simulator program for simulating a styrene plant where the same styrene prefractionation system is directly upstream of the styrene finishing system. The inventive styrene finishing system is that illustrated in FIG. 2. The comparative styrene finishing system is that illustrated in FIG. 2 but without the residue recycle reboiler 120 and residue recycle drum 124 and associated streams. Rather, in the comparative styrene finishing system, the residue stream 114 from the residue flash drum 264 is recycled back to the styrene prefractionation system. Each simulation has the following parameters:

- in all cases, the simulation produced 62,500 kg/hr of styrene monomer product, which has 15.6 kg/hr of AMS;
- the composition of the crude styrene from the reaction section of the styrene unit is typical for styrene plant operation;
- the flow ratio of the liquid from the residue flash drum 264 for the recycle stream (i.e., residue stream 114 in the inventive example and the stream directly recycled back to the styrene prefractionation system in the comparative example) to the net heavy by-product stream 116 is varied according to Table 1 for different simulations;
- in all cases, the residue flash drum 264 is at 152°C and 152 mmHg absolute of pressure;
- in all cases, the distillation finishing column 240 has the same number of theoretical stages, feed stage, pressure profile, and condenser outlet temperature;
- in the inventive examples, the residue recycle drum 124 is also at 152°C and 152 mmHg absolute; and
- in the inventive examples, the overhead vapor stream 126 from the residue recycle drum 124 is fed to the next to bottoms stage of distillation finishing column 240, with the bottoms stage being the finishing column reboiler 254.

Table 1

| Case | Recycle to Residue Flowrate Ratio[1] | Residue Recycle Stream[2] Components (kg/hr) | | | Finishing System Reboiler Heat Duties (millions of kcal/hr) | | | |
|---|---|---|---|---|---|---|---|---|
| | | Styrene | AMS | DVB | Column Reboiler | Residue Reboiler | Recycle Reboiler | Total |
| Comparative Styrene Finishing System | | | | | | | | |
| 1 | 0 | n/a | n/a | n/a | 5.70 | 2.49 | n/a | 8.19 |
| 2 | 2 | 83 | 88.5 | 6.43 | 7.63 | 2.49 | n/a | 10.12 |
| 3 | 5 | 205 | 220.2 | 15.91 | 8.93 | 2.49 | n/a | 11.42 |
| Inventive Styrene Finishing System | | | | | | | | |
| 4[3] | 2 | 118 | 12.8 | 1.92 | 6.06 | 2.49 | 0.16 | 8.71 |
| 5[4] | 2 | 116 | 8.7 | 1.40 | 5.89 | 2.48 | 0.23 | 8.60 |
| 6[5] | 2 | 114 | 6.6 | 1.08 | 5.78 | 2.48 | 0.31 | 8.57 |
| 7[6] | 5 | 303 | 17.5 | 2.87 | 5.86 | 2.47 | 0.77 | 9.10 |

[1] Flowrate of the residue stream 114 divided by the flowrate of the net heavy by-product stream 238. For a ratio of zero, residue stream 114 is absent.
[2] The residue stream 114 in the inventive examples. The residue stream 114 is directly recycled back to the styrene prefractionation system in the comparative examples.
[3] 2 wt% of styrene-rich stream 102 is diverted via the second portion 106 (or slip stream) of the residue recycle reboiler 120.
[4] 3 wt% of styrene-rich stream 102 is diverted via the second portion 106 of the residue recycle reboiler 120.
[5] 4 wt% of styrene-rich stream 102 is diverted via the second portion 106 of the residue recycle reboiler 120.
[6] 10 wt% of styrene-rich stream 102 is diverted via the second portion 106 of the residue recycle reboiler 120.

Case 1 is the styrene finishing system operating with no residue recycle. Case 2 is the operation with a 2.0 recycle to net residue ratio, which allows for the net feed of polymerization retarder to be reduced approximately in half relative to having no residue recycle. Case 3 has a residue recycle ratio of 5.0, which allows the retarder dosage to be about half of that in Case 2 or about one quarter of that in Case 1.

In all of the simulations summarized in the table, the crude styrene fed to the styrene prefractionation system has 59.8 kg/hr of AMS, but because of the recycled residue, the feed to the finishing column in Case 2 has 148.3 kg/hr of AMS (59.8 plus 88.5 from the table). Consequently, the styrene finishing system has to process almost 2.5 times as much of this component for Case 2 compared to Case 1 because of the residue recycle. This increase in AMS and in the other $C_9$ aromatics present raises the combined duty of the two reboilers in the comparative styrene finishing system by 24 percent (from 8.19 to 10.12 million kcal/hr). Increasing the ratio to 5.0 in Case 3 raises the combined reboiler heat requirement by nearly 40 percent relative to operation with no recycle.

Since DVB is not volatile enough to appreciably get to the styrene monomer product, whatever DVB does not react in the process, all ultimately ends up in the net residue. If the amount of DVB reacting is assumed to be negligible, then a recycle ratio of 2.0 triples (2+1) the amount of DVB in the upstream equipment and in the feed to the styrene finishing system. With a residue recycle ratio of 5.0, the DVB concentration goes up by a factor of six relative to operation with no recycle. It takes very little of this crosslinking agent, DVB, to create severe fouling. Consequently, Case 2 is the most likely of the three comparative examples to be implemented.

In Case 4, 2.0 percent of the styrene-rich feed coming into the system was diverted from the finishing column feed to the residue recycle reboiler and residue recycle drum, and the split ratio of liquid from the residue flash drum is 2.0 recycle to net residue. In most distillations with the feed stage part way up the column, putting part of the column feed to the bottom of the column would result in significantly higher reboiler duties, which reduces the likelihood of implementation. However, in Case 4, the combined reboiler duty for the styrene finishing system, including the new residue recycle reboiler, actually decreases by about 14 percent, which is notable when taking into account that more than half of the combined reboiler duty is needed just to boil the styrene product overhead and less than half of the combined reboiler duty is used to get the column reflux overhead. The styrene finishing system feed is typically well over 90 percent styrene, and putting some of this flow to the bottom of the column does make the separation more difficult because an overwhelming percentage of the styrene needs to go overhead, but as noted in the table, putting 2.0 percent of this feed stream into the recycle residue reboiler and drum radically reduces the amount of AMS and the other $C_9$ aromatics in the residue recycle back to the styrene prefractionation system, which makes the finishing system separation easier and lowers the net heat input required. Thus, putting part of the distillation feed to the bottom of the column, which is contrary to conventional wisdom in distillation practice, has a surprisingly beneficial effect.

Case 4 also lowers the flow of DVB in the recycle residue by 70 percent (6.43 to 1.92 kg/hr) relative to Case 2. In the styrene finishing system feed, the DVB flow is reduced by 47 percent. Consequently, the risk of serious fouling is reduced with Case 4 relative to Case 2.

The flow of styrene increases in the residue recycle in Case 4, and that styrene has to be repurified in the upstream column(s), but the increase is only 35 kg/hr, which is only 0.05 percent of the styrene flowing through the styrene prefractionation and styrene finishing systems. The extra reboiler duty in the upstream columns is in the round-off of the reboiler duties in the table, and thus, is negligible. Since the stripping process in the residue recycle reboiler and drum reduces the concentrations of the $C_9$ and $C_{10}$ aromatics in the recycle, there is the risk that the residue recycle stream becomes excessively viscous since the smaller molecules are keeping the viscosity of this polymer solution lower. Viscosity is a primary reason why, in commercial plant operation, styrene is typically left in the residue on the percent level, even though it is a yield loss. Consequently, there is some benefit in terms of pumpability and residue recycle reboiler heat transfer to have a higher concentration of styrene in the liquid when the $C_9$'s and $C_{10}$'s are partially stripped out.

Cases 5 and 6 are the same as Case 4 except the percentages of styrene-rich feed diverted to the residue recycle reboiler and drum are 3.0 and 4.0, respectively. At the costs of larger recycle residue reboilers and drums, these cases further reduce the combined finishing system reboiler duty, due to lower recycle flows of $C_9$ aromatics, and the recycle flow of DVB. Although equipment has been added for Cases 4 to 6, the added items are small relative to the rest of the styrene finishing system, as evidenced by the reboiler duties. Accordingly, the investment savings in the finishing column, finishing column condenser, and finishing column reboiler due to the addition of the residue recycle reboiler and drum may result in a net investment savings.

Case 7 is the same as the Cases 4 to 6 except that the split ratio is increased from 2.0 to 5.0, which allows the dosage of retarder to the styrene prefractionation system to be approximately halved relative to Cases 2, 4, 5, and 6. Not only does this reduction in dosage reduce the cost of supplying the retarder, but since the net residue is normally burned for its fuel value, the reductions in the dosages of inhibitors potentially reduces the emissions of nitrogen oxides to the environment. To effectively strip the added AMS and DVB from the recycle residue, the portion of finishing system feed diverted to the residue recycle reboiler and drum is increased to 10.0 percent. As shown in the table, the combined finishing system reboiler duty and DVB flow in the recycle both increase relative to the previous invention embodiments, but these values are much closer to those in Cases 4 to 6 than they are to those in Case 2. Since the DVB recycled is less than half of what it is in Case 2, increasing the recycle ratio from 2.0 to 5.0 becomes a viable option; whereas, without the residue recycle reboiler and drum, it is arguably not.

The inclusion of the residue recycle reboiler and drum in the styrene finishing system with different arrangements of equipment upstream of the residue recycle reboiler and drum may result in similar benefits to said styrene finishing systems.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein may be combined with any other examples to yield further examples.

**Claims**

1.  A system comprising:

    a styrene finishing system configured to receive a first portion of a styrene-rich stream from a styrene prefractionation system and produce a styrene monomer product, a residue stream, and a net heavy by-product stream;
    a residue recycle processor configured to process a second portion of the styrene-rich stream from the styrene prefractionation system and the residue stream from the styrene finishing system to produce an overhead vapor stream and a residue recycle stream; and
    wherein the styrene finishing system is further configured to receive the overhead vapor stream; and
    wherein the styrene prefractionation system is configured to receive the residue recycle stream from the residue recycle processer.

2.  The system of claim 1, wherein the residue recycle stream comprises 15 wt% or less of styrene and 0.10 wt% or greater of a polymerization inhibitor.

3.  The system of any preceding claim, wherein the second portion of the styrene-rich stream comprises 90 wt% or greater of styrene.

4.  The system of any preceding claim, wherein the residue recycle processor comprises a residue recycle reboiler and a residue recycle drum, wherein the residue recycle reboiler is configured to receive and heat the second portion of the styrene-rich stream from the styrene prefractionation system and the residue stream from the styrene finishing system as a combined stream to produce a partially vaporized stream, wherein the residue recycle drum is configured to receive and separate the partially vaporized stream to produce the overhead vapor stream and a bottoms liquid stream, and wherein at least a portion of the bottoms liquid stream is the residue recycle stream.

5.  The system of claim 4, wherein the system is further configured to mix a portion of the bottoms liquid stream with the residue stream and/or the second portion of the styrene-rich stream upstream of the residue recycle reboiler.

6.  The system of any preceding claim, wherein the styrene finishing system comprises one or more distillation finishing columns, one or more reboilers, and one or more drums.

7.  The system of any preceding claim, wherein the overhead vapor stream is a first overhead vapor stream, wherein the bottoms liquid stream is a first bottoms liquid stream, and wherein the styrene finishing system comprises:

    a distillation finishing column configured to receive the first portion of the styrene-rich stream from the styrene prefractionation system and the first overhead vapor stream from the residue recycle processor and to produce a second overhead vapor stream and a second bottoms liquid stream; and
    a residue reboiler and a residue flash drum in series configured to receive the second bottoms liquid stream and to produce a third overhead vapor stream and a third bottoms liquid stream, wherein the system is configured to split the third bottoms liquid stream to produce the residue stream and the net heavy by-product stream.

8.  A method comprising:

    purifying a first portion of a styrene-rich stream from a styrene prefractionation system in a styrene finishing system to produce a styrene monomer product, a residue stream, and a net heavy by-product stream;
    separating at least a portion of the residue stream using a second portion of the styrene-rich stream as a stripping agent to yield an overhead vapor stream and a bottoms liquid stream;

recycling the overhead vapor stream to the styrene finishing system; and
recycling at least a portion of the bottoms liquid stream as a residue recycle stream to the styrene prefractionation system.

9. The method of claim 8, wherein the residue stream comprises 15 wt% or less of styrene and 0.10 wt% or greater of a polymerization inhibitor.

10. The method of claim 8 or 9, wherein the second portion of the styrene-rich stream comprises 90 wt% or greater of styrene.

11. The method of any one of claims 8-10, wherein the second portion of the styrene-rich stream is up to 25 wt% of the styrene-rich stream.

12. The method of any one of claims 8-11, wherein a percent reduction of alpha-methylstyrene in the residue recycle stream compared to the residue stream is 50% or greater.

13. The method of any one of claims 8-12, wherein a percent reduction of divinylbenzene in the residue recycle stream compared to the residue stream is 40% or greater.

14. The method of any one of claims 8-13, wherein a weight ratio of the residue stream to the net heavy by-product stream is 0.5:1 to 10:1.

15. The method of any one of claims 8-14, wherein a weight ratio of the second portion of the styrene-rich stream to the residue stream is 0.1:1 to 10:1.

*FIG. 1*

FIG. 2

200

EP 4 741 368 A1

13

300

**301**
purifying a first portion of a styrene-rich stream from a styrene prefractionation system in a styrene finishing system to yield a styrene monomer product stream and two residue streams

**302**
heating then separating at least a portion of one of the residue stream using a second portion of the styrene-rich stream as a stripping agent to yield a overhead vapor stream and a bottoms liquid stream

**303**
recycling the overhead vapor stream to the styrene finishing system

**304**
recycling at least a portion of the bottoms liquid stream as a residue recycle stream to the styrene prefractionation system

*FIG. 3*

Europäisches
Patentamt

European
Patent Office

Office européen
des brevets

# EUROPEAN SEARCH REPORT

**Application Number**

EP 24 30 6888

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2024/020230 A1 (T EN PROCESS TECH INC [US]) 25 January 2024 (2024-01-25) * page 9, line 15 - page 13, line 16; figure 2 * * claims 1-19 * | 1-15 | INV. C07C7/04 C07C15/46 |
| A | US 9 902 667 B2 (TECHNIP PROCESS TECH INC [US]) 27 February 2018 (2018-02-27) * claim 1; figures 1,2 * * page 7, column 4 - page 8, column 5 * | 1-15 | |

**TECHNICAL FIELDS SEARCHED (IPC)**

C07C

The present search report has been drawn up for all claims

| Place of search | Date of completion of the search | Examiner |
|---|---|---|
| The Hague | 3 July 2025 | Kövecs, Monika |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 30 6888

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2025

| Patent document cited in search report | | Publication date | Patent family member(s) | | Publication date |
|---|---|---|---|---|---|
| WO 2024020230 | A1 | 25-01-2024 | CN | 119744255 A | 01-04-2025 |
| | | | EP | 4547632 A1 | 07-05-2025 |
| | | | KR | 20250038737 A | 19-03-2025 |
| | | | TW | 202404929 A | 01-02-2024 |
| | | | US | 2024026231 A1 | 25-01-2024 |
| | | | WO | 2024020230 A1 | 25-01-2024 |
| US 9902667 | B2 | 27-02-2018 | BR | 112015014290 A2 | 11-07-2017 |
| | | | CN | 104903280 A | 09-09-2015 |
| | | | KR | 20150088892 A | 03-08-2015 |
| | | | KR | 20170098328 A | 29-08-2017 |
| | | | US | 2015336859 A1 | 26-11-2015 |
| | | | WO | 2014098816 A1 | 26-06-2014 |

EPO FORM P0459

For more details about this annex : see Official Journal of the European Patent Office, No. 12/82